# EUROPEAN PATENT APPLICATION

(11) **EP 2 620 172 A1**
(43) Date of publication of application: **31.07.2013**
(21) Application number: 11826722.8
(22) Date of filing: 06.09.2011
(51) Int. Cl.: A61M 5/158, A61M 5/28

(54) **METHOD OF ASSEMBLING MEDICAMENT INJECTION DEVICE**

(30) Priority: 24.09.2010 JP 2010213343
(71) Applicant: Terumo Kabushiki Kaisha, Tokyo 151-0072 (JP)
(72) Inventor: TACHIKAWA Kouichi, Fujinomiya-shi Shizuoka 418-0004 (JP); OGAWA Junichi, Nakakoma-gun Yamanashi 409-3853 (JP)
(74) Representative: TBK
(86) International application number: PCT/JP2011/070212
(87) International publication number: WO 2012/039276

(57) **Abstract**

Disclosed is a method of assembling a medicament injection device (1) including the following steps: a step of integrally fixing a needle tube (3) to a needle hub (4) by high-frequency welding; a step of radiation sterilizing the needle tube (3) and the needle hub (4); a step of subjecting a container (2) to be filled with a medicament (M) to a sterilization treatment which involves generation of heat; a step of aseptically transporting the needle hub (4) which has been subjected to the radiation sterilization into an isolator device (H); a step of fixing the needle hub (4) to the container (2) in a sterile environment in the isolator device (H); and a step of filling the container (2) fixing the needle hub (4) with the medicament (M) in the sterile environment.

## Description

### Technical Field

The present invention relates to a method of assembling a medicament injection device in which a needle tube and a needle hub are integrally fixed to each other by high-frequency welding.

### Background Art

In recent years, medicament injection devices in which a syringe as a container is preliminarily filled with a medicament have come to be used frequently (see, for example, Patent Document 1). In the medicament injection device described in Patent Document 1, a double headed needle held by a needle hub is attached to an outer tube preliminarily filled with a medicament.

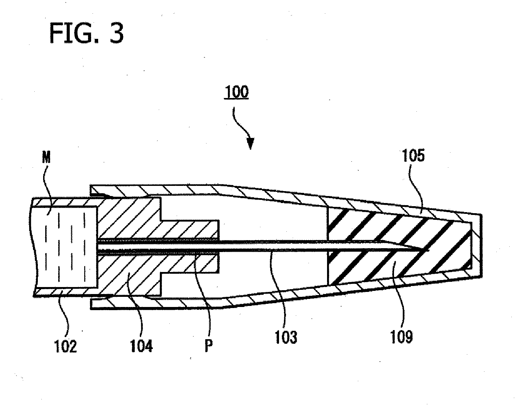

Besides, prefilled syringes having a needle tube have also been used. Now, a conventional prefilled syringe with a needle tube will be described below, referring to FIG. 3.
FIG. 3 is a sectional view showing a conventional prefilled syringe.

As shown in FIG. 3, the prefilled syringe 100 includes an outer tube 102 as a container for containing a medicament M, a needle tube 103, a needle hub 104, a cap 105, and a pusher (plunger) which is not shown. The outer tube 102 is formed in a substantially cylindrical shape opening at both ends thereof. At an axial-directionally one end portion of the outer tube 102, the needle hub 104 is provided integrally. Besides, the needle tube 103 is integrally fixed to the needle hub 104 by high-frequency welding. Therefore, the needle tube 103 and the needle hub 104 are welded to each other at a weld P.

In addition, a gasket as the pusher which is not shown is put into an axial-directionally other end portion of the outer tube 102. Furthermore, the cap 105 is detachably mounted so as to cover a needle tip of the needle tube 103 and the needle hub 104. Besides, a sealing member 109 is provided in a tip portion of the inside of the cap 105. The needle tip of the needle tube 103 is being sealed by the sealing member 109.

In general, the needle tube and the needle hub are fixed to each other by use of the high-frequency welding, which is a fixing method which does not produce an influence on the medicament. In addition, for keeping sterility and stability and the like of the medicament, it has been necessary to subject the container to a sterilization treatment such as AC (autoclave) sterilization and EOG (ethylene oxide gas) sterilization. Here, the AC sterilization and EOG sterilization involve generation of heat, since they are each a treatment method in which the body to be treated (the object of treatment) is exposed to high-temperature steam or gas. Besides, when the body having undergone the high-frequency welding is subjected to a sterilization treatment which involves generation of heat, the weld is again heated, so that the strength of the weld may be lowered.

### Prior Art Document

### Patent Document

Patent Document 1: Japanese Patent Laid-open No. Hei 8-117334

### Summary of Invention

### Technical Problem

In the conventional medicament injection devices, however, the needle tube and the needle hub having been integrally fixed to each other by the high-frequency welding have also been subjected to the sterilization treatment which involves the generation of head, such as the AC sterilization and the EOG sterilization. Consequently, the conventional medicament injection devices have a problem that the strength of the weld may be lowered, since the needle tube and the needle hub having been integrally fixed to each other by the high-frequency welding are also subjected to the sterilization treatment which involves the generation of heat.

In consideration of the above-mentioned problem, it is an object of the present invention to provide a method of assembling a medicament injection device by which it is possible to prevent a lowering in the strength of a weld between a needle tube and a needle hub having been integrally fixed to each other by high-frequency welding.

### Technical Solution

In order to solve the above-mentioned problem and attain the object of the present invention, a method of assembling a medicament injection device according to the present invention includes the following steps (1) to (6) :
(1) integrally fixing a needle tube to a needle hub for holding the needle tube by high-frequency welding, and the needle tube having a needle tip capable of puncturing a living body;
(2) subjecting the needle tube and the needle hub fixing the needle tube to a radiation sterilizing treatment;
(3) subjecting a container to be filled with a medicament to a sterilization treatment which involves generation of heat;
(4) aseptically transporting the needle hub having been subjected to the radiation sterilization treatment into an isolator device;
(5) fixing the needle hub having been subjected to the radiation sterilization treatment to the container having been subjected to the sterilization treatment which involves the generation of heat in a sterile environment in the isolator device; and
(6) filling the container fixing the needle hub with the medicament in the sterile environment.

### Advantageous Effect

According to the method of assembling a medicament injection device of the present invention, the needle tube and the needle hub having been integrally fixed to each other by the high-frequency welding are subjected to the sterilization treatment by the radiation sterilization. This makes it possible to prevent the strength of the weld from being lowered due to heat.

### Brief Description of Drawings

[FIG. 1]

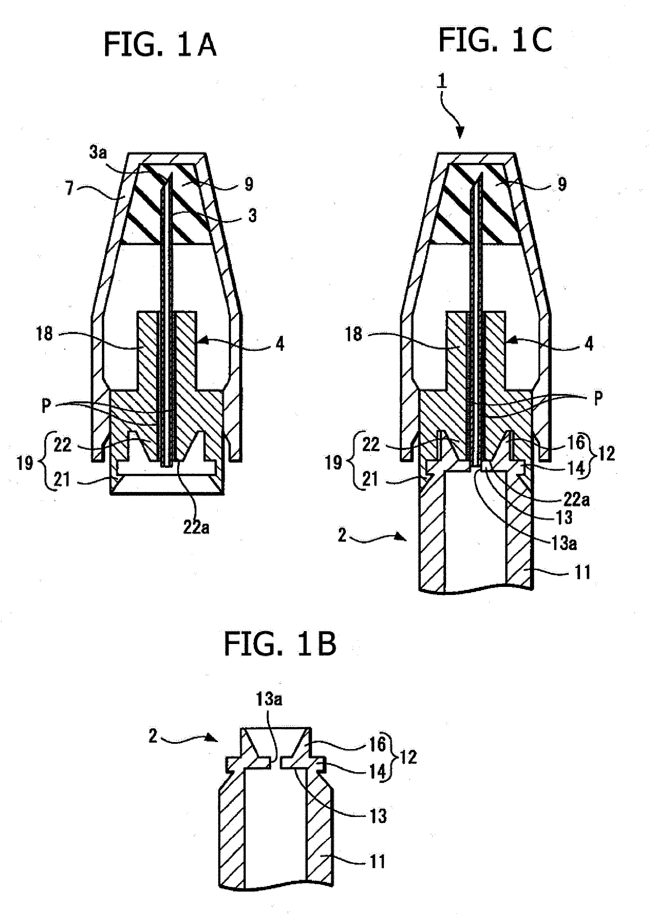

   FIG. 1 shows views for illustrating a method of assembling a medicament injection device according to the present invention, wherein FIG. 1(A) is a sectional view showing a needle tube and a needle hub, FIG. 1(B) is a sectional view showing a container, and FIG. 1(C) is a sectional view showing a condition where the needle hub and the container have been fixed to each other.
[FIG. 2]

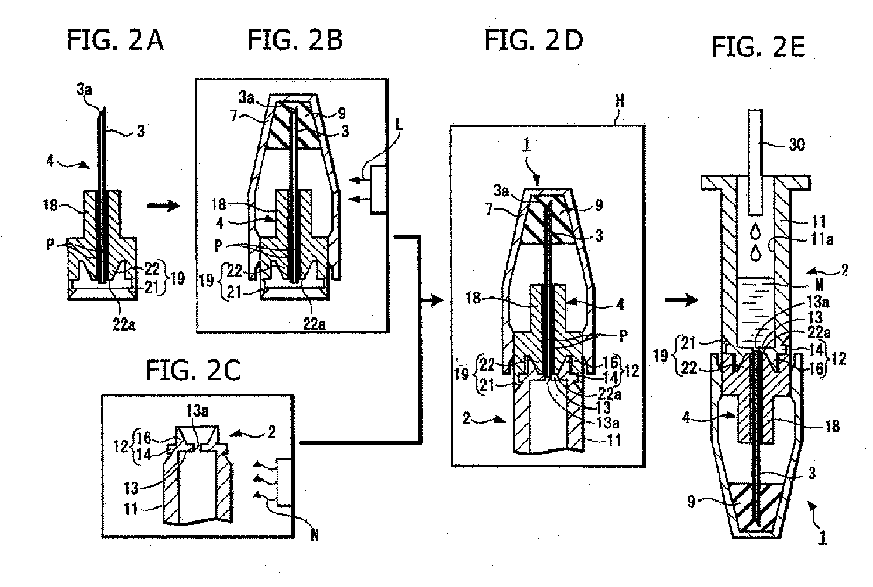

   FIG. 2 is a flow diagram illustrating an assembly procedure in a method of assembling a medicament injection device according to the present invention.
[FIG. 3]
   FIG. 3 is a sectional view showing a conventional prefilled syringe with needle.

### Mode for Carrying Out the Invention

Now, an embodiment (example of carrying-out mode) of a medicament injection device according to the present invention will be described below, referring to FIGS. 1 and 2. Incidentally, the members shown in common in the figures are denoted by the same reference symbols. In addition, the present invention is not to be restricted to the following embodiment. Incidentally, description will be made in the following order.
1. Configuration Example of Medicament Injection Device
2. Method of Assembling Medicament Injection Device

### 1. Configuration Example of Medicament Injection Device

First, an embodiment of a prefilled syringe based on application of the medicament injection device according to the present invention (hereinafter referred to as "this embodiment") will be described referring to FIG. 1.
FIG. 1 shows sectional views of a prefilled syringe according to this embodiment.

The prefilled syringe 1 in this embodiment is preliminarily filled with a medicament M to be administered into a living body. As shown in FIG. 1(C), the prefilled syringe 1 includes an outer tube 2 for accommodating the medicament M therein, a hollow needle tube 3 having a needle hole, a needle hub 4 for holding the needle tube 3, a cap 7, and a pusher (plunger) which is not shown.

The medicament M preliminarily accommodated in the prefilled syringe 1 may be any medicament that is ordinarily used as an injection product. Examples of the medicament M include protein medicaments such as antibodies, etc., peptide medicaments such as hormones, etc., nucleic acid medicaments, cell medicaments, blood derivatives, vaccines for prevention of various infectious diseases, carcinostatic agents, anesthetics, narcotics, antibiotics, steroid preparations, proteinase inhibitors, heparin, saccharide injections such as glucose, etc., electrolyte correction injections such as sodium chloride, potassium lactate, etc., vitamin preparations, lipid emulsions, contrast media, and stimulants.

### [Outer Tube]

First, the outer tube 2 will be described.
The outer tube 2 representing a container in the present invention has a syringe body 11, and an outer tube-side fixing section 12 continuous with the syringe body 11. The syringe body 11 is formed in a hollow substantially cylindrical shape, and is opened at both axial-directional ends thereof. On axial-directionally one side of the syringe body 11, the outer tube-side fixing section 12 is formed continuously. In addition, a tube hole 11a of the syringe body 11 is filled with the medicament M (See FIG. 2(E).), and a gasket as the pusher (plunger) is put into a position from the axial-directionally other side of the syringe body 11.

Furthermore, at axial-directionally one end of the syringe body 11 is formed an inner flange section 13 which projects radially inward. In a substantially central area of the inner flange section 13 is provided a discharge port 13a to discharge the medicament.

The outer tube-side fixing section 12 has an engaging receptor 14 representing a container-side engaging receptor, and a fitting receptor 16. The engaging receptor 14 is an outer flange projecting radially outward from an outer circumferential surface of the syringe body 11. With the engaging receptor 14, an engaging claw 21 of the needle hub 4 which will be described later is engaged. In addition, the fitting receptor 16 is disposed at the axial-directionally one end of the syringe body 11. The fitting receptor 16 is a tapered recess of which the diameter (inner diameter) decreases along the direction from the tip thereof toward the syringe body 11. Besides, into the fitting receptor 16, a fitting projection 22 of the needle hub 4 which will be described later is taper fitted.

Examples of a material of the outer tube 2 include various resins such as polyvinyl chloride, polyethylene, polypropylene, cyclic polyolefins, polystyrene, poly(4-methylpentene-1), polycarbonates, acrylic resins, an acrylonitrile-butadiene-styrene copolymer, polyesters such as polyethylene terephthalate, etc., a butadiene-styrene copolymer and polyamides (e.g., nylon 6, nylon 6·6, nylon 6·10, nylon 12). Among these, preferred are such resins as polypropylene, cyclic polyolefins, polyesters, and poly(4-methylpentene-1), from the viewpoint of ease of molding. Incidentally, it is preferable that the material of the outer tube 2 is substantially transparent, for securing inside visibility.

Incidentally, while the example wherein the syringe body 11 of the outer tube 2 has the hollow substantially cylindrical shape has been described in this embodiment, the shape of the outer tube 2 may be a hollow tetragonal prism or hexagonal prism.

### [Needle Tube]

Now, the needle tube 3 will be described.
As shown in FIG. 1(A), as the needle tube 3, a needle tube with a size (outside diameter: 3.4 to 0.1 mm) of 10 to 36 gauge, preferably a size (outside diameter: 1.6 to 0.2 mm) of 14 to 33 gauge, according to the ISO medical needle tube standard (ISO 9626: 1991/Amd. 1:2001(E)) is used. The needle tube 3 is provided at one end thereof with a cutting edge surface 3a for making the needle tip have an acute angle.

A material of the needle tube 3 may be, for example, stainless steel; however, this is not restrictive, and other metals such as aluminum, aluminum alloys, titanium, and titanium alloys can also be used as the material. In addition, the needle tube 3 is not limited to a straight needle, and may be a double edged needle or a tapered needle which is tapered at least at a part thereof. It suffices for the tapered needle to have a proximal portion greater in diameter than a needle tip portion and to have an intermediate portion of a tapered structure. Besides, the sectional shape of the needle tube 3 is not restricted to a circle but may be a polygon such as a triangle.

### [Needle Hub]

Now, the needle hub 4 will be described.
As shown in FIG. 1(A), the needle hub 4 has a substantially cylindrical hub body 18, and a hub-side fixing section 19. Examples of a material of the needle hub 4 include synthetic resins such as polycarbonate, polypropylene, and polyethylene. In addition, the needle tube 3 and the hub body 18 are integrally fixed to each other by the high-frequency welding. Specifically, the needle tube 3 is fixed to the hub body 18 through a weld P. Besides, the needle tip of the needle tube 3 is protruding from axial-directionally one end of the hub body 18.

The hub-side fixing section 19 is provided at the axial-directionally other side of the hub body 18. The hub-side fixing section 19 has an engaging claw 21 as a hub-side engaging claw, and a fitting projection 22. The engaging claw 21 is protruding from the outer circumference of the axial-directionally other end of the hub body 18. In addition, a tip of the engaging claw 21 is formed in a hook shape.

The fitting projection 22 is projecting from a substantially central portion of an end face on the axial-directionally other side of the hub body 18. This fitting projection 22 is formed in a tapered shape (roughly truncated conical shape) such as to be decreased in diameter toward the tip side. Besides, the other end of the needle tube 3 is protruding from an end face 22a of the fitting projection 22. In addition, when the fitting projection 22 and the fitting receptor 16 are put into taper fit, the center of the end face of the fitting projection 22 coincides with the axis of the syringe body 11.

Incidentally, the shape of the hub body 18 of the needle hub 4 is not restricted to the cylindrical shape; for example, the shape may be a polygonal tubular shape such as a tetragonal prism or hexagonal prism provided with a tube hole in the center thereof. While the example wherein the outer tube 2 is provided with the engaging receptor 14 and the needle hub 4 is provided with the engaging claw 21 has been described in this embodiment, a configuration may be adopted wherein the outer tube 2 is provided with an engaging claw and the needle hub 4 is provided with an engaging receptor for engagement with the engaging claw. Furthermore, while the example wherein the outer tube 2 is provided with the fitting receptor 16 and the needle hub 4 is provided with the fitting projection 22 has been described in this embodiment, a configuration may be adopted wherein the outer tube 2 is provided with a fitting projection and the needle hub 4 is provided with a fitting receptor for taper fit with the fitting projection.

In addition, while the example wherein the needle hub 4 and the outer tube 2 are fixed to each other by engagement between the engaging claw 21 and the engaging receptor 14 has been described, this is not restrictive. For example, a configuration may be adopted wherein the needle hub 4 is provided with a threaded groove whereas the outer tube 2 is provided with a screw part for screw engagement with a threaded groove, and the threaded groove and the screw part are engaged with each other, thereby fixing the needle hub 4 to the outer tube 2.

### [Cap]

Now, the cap 7 will be described.
As shown in FIGS. 1(A) and 1(C), the cap 7 is formed in a hollow substantially truncated conical shape. The cap 7 is opened at axial-directionally one end and closed at the other end. In addition, the cap 7 is detachably attached to the needle hub 4 so as to cover the needle tip of the needle tube 3 and the hub body 18 of the needle hub 4. Incidentally, the cap 7 may be so formed as to cover not only the needle hub 4 but also an end portion of the outer tube 2.

In addition, the cap 7 is provided therein with a sealing member 9 at a tip portion which is on the other end side. The sealing member 9 seals the needle tip of the needle tube 3 when the cap 7 is attached to the needle hub 4. This ensures that leakage of the medicament M from the needle tip of the needle tube 3 can be prevented from occurring when the outer tube 2 is filled with the medicament M or during storage.

Examples of a material of the sealing member 9 include various rubber materials such as natural rubber, butyl rubber, isoprene rubber, butadiene rubber, styrenebutadiene rubber, silicone rubbers, and isobutylene rubber, various thermoplastic elastomers based on polyurethane, polyester, polyamide, olefin or styrene, and an elastic material such as mixtures of them.

### 2. Method of Assembling Medicament Injection Device

Now, a method of assembling the prefilled syringe 1 in this embodiment which is configured as above will be described below, referring to FIGS. 2(A) to 2(E).

First, as shown in FIG. 2(A), the needle tube 3 and the needle hub 4 are integrally fixed to each other by high-frequency welding. Next, as shown in FIG. 2(B), the needle tube 3 and the needle hub 4 integrally fixed to each other and the cap 7 are subjected to so-called radiation sterilization treatment in which they are irradiated with radiation L such as γ-rays and electron beams. By thus subjecting the needle tube 3 and the needle hub 4, having been integrally fixed to each other by the high-frequency welding, to the radiation sterilization treatment in which heat is not generated, it is possible to prevent the strength of the weld P from being lowered.

In addition, as shown in FIG. 2(C), the outer tube 2 is subjected to a sterilization treatment which generates heat N, such as AC (autoclave) sterilization and EOG (ethylene oxide gas) sterilization.

Incidentally, while the example in which the cap 7 is subjected to the radiation sterilization treatment has been described in this embodiment, the cap 7 may be subjected to the sterilization treatment which generates the heat, such as the AC (autoclave) sterilization and the EOG (ethylene oxide gas) sterilization, like the outer tube 2, since the cap 7 has not been subjected to the high-frequency welding. Besides, with the cap 7 also subjected to the sterilization treatment, the space inside the cap 7 can be put into an aseptic state. In addition, by covering the needle tube 3 with this cap 7, it is possible to prevent particulates from adhering to the needle tip of the needle tube 3 during the assembling operation, and to maintain the aseptic state of the needle tip of the needle tube 3.

Subsequently, the needle tube 3 and the needle hub 4 having undergone the sterilization treatment and the outer tube 2 are aseptically transported into an isolator device H. Then, as shown in FIG. 2(D), the hub-side fixing section 19 of the needle hub 4 having undergone the sterilization treatment and the outer tube-side fixing section 12 of the outer tube 2 are fixed to each other in the sterile environment in the isolator device H. Specifically, the fitting projection 22 of the hub-side fixing section 19 is inserted into the fitting receptor 16 of the outer tube-side fixing section 12. Then, the engaging claw 21 of the hub-side fixing section 19 is put into engagement with the engaging receptor 14 of the outer tube-side fixing section 12.

When the engaging claw 21 and the engaging receptor 14 are engaged together, the fitting projection 22 is pushed into the fitting receptor 16, and the fitting projection 22 and the fitting receptor 16 are taper fitted to each other. With the fitting projection 22 and the fitting receptor 16 thus taper fitted to each other, the side surface of the fitting projection 22 and the inner circumferential surface of the fitting receptor 16 are put into secure contact with each other in a liquid-tight manner.

Here, in the case where the needle hub 4 and the outer tube 2 are connected by only the taper fit between the fitting projection 22 and the fitting receptor 16, the dimensional accuracy of the fitting projection 22 and the fitting receptor 16 must be set high. In addition, in the case where the needle hub 4 and the outer tube 2 are connected by only the engaging claw 21 and the engaging receptor 14, there is a need for a member for securing liquid-tightness between the outer tube 2 and the needle hub 4, such as a seal member.

In this embodiment, however, the connection between the needle hub 4 and the outer tube 2 is achieved by joint use of both the engagement between the engaging claw 21 and the engaging receptor 14 and the taper fit between the fitting projection 22 and the fitting receptor 16. This makes it possible to not only adopt a low dimensional accuracy of the fitting projection 22 and the fitting receptor 16 but also omit a member for securing liquid-tightness between the outer tube 2 and the needle hub 4, such as a seal member.

Next, as shown in FIG. 2(E), a nozzle 30 to eject a medicament M is inserted into the tube hole 11a of the syringe body 11. Then, the nozzle 30 is operated to inject the medicament M into the tube hole 11a. Therefore, the tube hole 11a of the syringe body 11 is filled with the medicament M. In this instance, the needle tip of the needle tube 3 is being sealed with the sealing member 9 provided in the cap 5. This ensures that leakage of the medicament M through the needle tip of the needle tube 3 can be prevented from occurring at the time of filling with the medicament M. Incidentally, the step of filling with the medicament M is also carried out in a sterile environment in the isolator device H. Then, the gasket constituting the pusher is put into the tube hole 11a of the syringe body 11 filled with the medicament M. As a result, assembly of the prefilled syringe 1 is completed.

Incidentally, as a method for fixing the needle hub 4 to the outer tube 2, welding by use of laser, heat, high frequency wave or the like and adhesion fixing by use of an adhesive may be applied. In the cases of the welding and the adhesion fixing, however, a device for fixing is complicated and, therefore, a risk of a trouble generated in the device for fixing is increased. In addition, it is difficult to repair a troubled device in the sterile environment in the isolator. Therefore, there is a risk that a yield of the desired product may be lowered.

In view of this, therefore, in this embodiment, a mechanical fixing method involving the engagement between the engaging claw 21 and the engaging receptor 14 as well as the taper fit between the fitting projection 22 and the fitting receptor 16 is employed as the method for fixing the needle hub 4 to the outer tube 2. Such a mechanical fixing method can be carried out by use of a simple device which is low in risk of troubles, and can therefore enhance the yield of the desired product.

Incidentally, the present invention is not restricted to the embodiment described above and illustrated in the drawings, and various modifications are possible within the scope of the invention as defined in the claims.

### Industrial Applicability

The present invention can be utilized in assembling a medicament injection device which includes a needle tube and a needle hub.

### Explanation of Reference Symbols

1 Prefilled syringe (medicament injection device), 2 ... Outer tube (container),
3 Needle tube,
4 Needle hub, 7 ... Cap, 9 ... Sealing member, 12 ... Outer tube-side fixing section,
14 Engaging receptor (container-side engaging receptor), 16 ... Fitting receptor, 18 ... Hub body, 19 ... Hub-side fixing section, 21 ... Engaging claw (hub-side engaging claw), 22 ... Fitting projection, 30 ... Nozzle, M ... Medicament, P ... Weld, H ... Isolator device

## Claims

1. A method of assembling a medicament injection device, comprising:
integrally fixing a needle tube to a needle hub for holding the needle tube by high-frequency welding, and the needle tube having a needle tip capable of puncturing a living body;
subjecting the needle tube and the needle hub fixing the needle tube to a radiation sterilization treatment;
subjecting a container to be filled with a medicament to a sterilization treatment which involves generation of heat;
aseptically transporting the needle hub having been subjected to the radiation sterilization treatment into an isolator device;
fixing the needle hub having been subjected to the radiation sterilization treatment to the container having been subjected to the sterilization treatment which involves the generation of heat in a sterile environment in the isolator device; and
filling the container fixing the needle hub with the medicament in the sterile environment.

2. The method of assembling the medicament injection device according to claim 1,
wherein the fixing the needle hub to the container is carried out by a mechanical fixing method.

3. The method of assembling the medicament injection device according to claim 2,
wherein the needle hub includes a hub-side engaging claw or a hub-side engaging receptor, and
the container includes a container-side engaging receptor for engagement with the hub-side engaging claw or a container-side engaging claw for engagement with the hub-side engaging receptor.

4. The method of assembling the medicament injection device according to claim 2,
wherein the needle hub and the container are fixed to each other in a liquid-tight manner by taper fit.

5. The method of assembling the medicament injection device according to claim 1 further comprising
putting a gasket into the container filled with the medicament, in the sterile environment.
